# EUROPEAN PATENT APPLICATION

(11) **EP 4 062 921 A1**
(43) Date of publication of application: **28.09.2022**
(21) Application number: 22164405.7
(22) Date of filing: 25.03.2022
(51) Int. Cl.: A61K 36/185, A61K 36/288, A61K 36/738, B01D 1/00

(54) **EXTRACT PRODUCED BY MEANS OF A SOLVENT AND METHOD FOR PRODUCING THE EXTRACT**

(30) Priority: 25.03.2021 FI 20215341
(71) Applicant: Finthinktur Oy, 78310 Varkaus (FI)
(72) Inventor: KAUNISTO, Harri, 78310 Varkaus (FI)
(74) Representative: Salomäki, Juha Kari Ensio

(57) **Abstract**

The object of the invention is an extract from organic starting materials produced by means of a solvent and a method for producing the extract. In the method, alkaline ash extract of botanical or fungal origin is used as a solvent, as a consequence of which the finished extract contains components contained in the ash extract used as a solvent.

## Description

### FIELD OF THE INVENTION

The object of the invention is an extract as defined in the preamble of claim 1 and a method as defined in the preamble of claim 7 for producing the aforementioned extract, and also the use of ash extract as a solvent as presented in claim 15.

Extraction is a chemical separation method in which the desired substance can be separated from a solution on the basis of the solubility characteristics of the substance. Extraction can be used either to recover substances, or to separate or clean them. According to what is known in the art, in use are many different extraction methods and different extraction processes that have their own intended uses. In an extraction process, a suitable solvent is selected e.g. according to the substance to be recovered.

Water is most widely used as a solvent, which dissolves water-soluble substances well. Also widely used are alcohol solvents or water-alcohol solvents, which dissolve greases and oils well. In extraction, it is endeavored to recover their desired substances or to raise the concentration of them.

The invention relates to an extract product, i.e. more concisely an extract, which contains components from the solvent, e.g. from the ash extract used as a solvent, such as from birch ash extract. In addition, the invention relates to an extraction method wherein ash extract, such as birch ash extract, is used as a solvent. Materials to be extracted i.e. used in producing an extract, hereinafter starting materials, are various biogenic materials, such as parts of plants or fungi.

Birch ash extract is a strongly alkaline substance, which is traditionally used for the treatment of various bodily disorders. By using water as a solvent, birch ash extract, which is also called ash lye, is produced from birch ash. Birch ash extract is conventionally made by boiling birch ash in water and by filtering the loose ash out of the extract obtained. In addition to water, the solvent can also be part alcohol.

### BACKGROUND OF THE INVENTION

In the extraction method according to the invention, it is endeavored to recover the desired substances better than in extraction methods already known in the art. This is brought about by using alkaline ash extract, e.g. birch ash extract, as a solvent of the starting materials, said solvent also containing useful substances obtained from the ash.

Preferably in the method according to the invention, birch ash extract is used as a solvent to extract from various starting materials important and desired bioactive compounds, such as phenol compounds, into the extract. The extracts obtained contain minerals that are in the birch ash extract, said minerals being both macrominerals and microminerals, i.e. trace minerals, and which are 40-60 different minerals. One aim of the invention is to enhance the efficiency of the recovery of compounds contained in starting materials. Birch ash extract dissolves substances more effectively from the parts of plants and other starting materials than water or a water-alcohol solution.

By means of the method according to the invention a more alkaline extract is obtained, and alkaline birch ash extract dissolves compounds from starting materials more efficiently than the conventional solvents of water and/or alcohol. An alkaline solvent breaks down cell membranes and increases efficiency in, *inter alia,* the recovery of phenolic compounds.

With this method it is possible to obtain a strongly alkaline extract, which contains many desired substances in addition to the aforementioned 40-60 minerals contained in birch ash extract. Ash extract, such as birch ash extract, mixed with alcohol can also be used as a solvent, in which case the efficiency of recovering some substances, more particularly oils and greases, can be improved.

It is known in the art to extract bioactive compounds from biogenic starting materials. One method known in the art is disclosed in patent specification EP2793905B1. In the method, the bark of a tree belonging to the beech family, such as oak, is dissolved in water at 50 °C. However, not as many minerals are obtained in the extract produced in this way as are obtained by using the ash extract according to the invention as a solvent.

Specification US2009223000A1 discloses a method for manufacturing extract. In the method material of wood origin is dissolved first in an aqueous solution, in alcohol and/or in an alkaline solution for a period of 2-48 hours at room temperature. After this the temperature is raised to 60-120 °C. The alkali can be e.g. an aqueous solution of ash, i.e. *"water of ash (lye)".* In the method, 0.01-20 g (or even 50 g) of an alkali, such as water of ash, is used per liter of extraction solution. From this notation, it is not clear how much of the ash itself is in the extraction solvent because the specification does not mention how much ash is in the water of ash. Even if the aforementioned amount of dry ash, 0.01-20 g (or even 50 g) per liter of extraction solution, were placed directly into the extraction solvent, in which case the ash concentration would be a maximum 5 percent by mass (m-%), then the ash concentration and pH of the extraction solution of the specification would still be significantly lower than in the solution according to the invention. According to the specification, the extract solvent would contain a considerably smaller amount of minerals dissolved from the ash than in the solution according to the invention. The term *"water of ash (lye)"* cannot unambiguously be deduced to refer to the ash *extract* that is used in the method according to the invention. Neither is it clear from the specification how long the extraction is continued at a raised temperature.

### AIM OF THE INVENTION

Conventional extraction with water extracts water-soluble substances efficiently, but recovering greases with it is not efficient. A conventional water-alcohol solution improves the recovery of greases and oils in the extraction process. One aim of the present invention to efficiently recover selected substances and to produce an extract with alkalinity originating from the ash extract, e.g. birch ash extract, and containing trace ingredients of the birch ash extract.

The extract according to the invention is characterized by what is disclosed in the characterization part of claim 1. Correspondingly, the method according to the invention for producing the extract is characterized by what is disclosed in the characterization part of claim 7. Other embodiments of the invention are characterized by what is disclosed in the other claims. The invention is also characterized by what is disclosed in claim 15.

### BRIEF DESCRIPTION OF THE INVENTION

Preferably the extract according to the invention contains minerals from alkaline ash extract of plant origin, such as from birch ash extract, combined with components obtained from the lixiviated starting material. In the extraction method according to the invention alkaline ash extract made from ash of plant origin, such as birch ash, with various solvents and in various manners, such as by boiling in water, is used as a solvent. Approx. 6-25 % by mass of ash with respect to the amount of solvent is used for producing the ash extract to be used as a solvent.

The extract product according to the invention can also advantageously contain residues from the type of ash extract that has been produced by using birch sap as a solvent. In this case, in the extraction method according to the invention ash extract of plant origin, such as birch ash extract, is used as a solvent, which ash extract itself was produced from birch ash by using birch sap and/or a water-alcohol solution as a solvent. In this case, there are also components from birch sap, such as minerals, in the final extract product.

Preferably in the present invention the strong alkalinity of conventional ash extract, such as of birch ash extract, and of birch ash extract boiled from birch sap is utilized and also their good ability to dissolve the desired substances, e.g. phenolic compounds, from plants, fungi and other suitable starting materials. By adding alcohol in the extraction process to the ash extract that is the solvent, a very preservable extract, i.e. extract product, is obtained.

Preferably by means of the method according to the invention the health-promoting and/or health-improving substances of, *inter alia,* polypores or berries are dissolved from them with a solvent extracted from ash, such as birch ash, which solvent has high alkalinity.

When alcohol is added to the birch ash extract used as a solvent, or the extraction process is performed again after the ash solvent by using alcohol or an alcohol mixture as a solvent, extract products that are very preservable can be brought about by combining the extracts thus obtained.

### ADVANTAGES OF THE INVENTION

With the solution according to the invention an alkaline extract is obtained, in which are components of the ash extract used as a solvent in addition to the extracted substances. Preferably in the solution according to the invention the ash extract used as a solvent, such as birch ash extract, can be produced by boiling in birch sap. By replacing the conventional water or water-alcohol solution used as a solvent with birch sap in the production of ash extract, substances in the birch sap are obtained in the ash extract to be used as a solvent. As a solvent, the alkaline birch ash extract according to the invention can extract some substances better, and for that reason improves the yield of desired substances.

The extraction method according to the invention is suited to replace conventional extraction, in which water or a mixture of water and alcohol is used as a solvent.

### LIST OF FIGURES

In the following, the invention will be described in more detail with reference to the attached simplified diagrammatic drawings, wherein:
- Fig. 1: presents a general production method for the ash extract to be used as a solvent according to the invention;
- Fig. 2: presents a general production method for the extract product according to the invention;
- Fig. 3: presents one preferred method (A) according to the invention comprising distillation for producing extract; and
- Fig. 4: presents a second preferred method (B) according to the invention comprising distillation for producing extract.

Abbreviations in the figures:
- SM = starting material
- SO = solvent
- DR = distillation residue
- AE = ash extract
- P₀ = solvent production phase
- P = extract production phase

### DETAILED DESCRIPTION OF THE INVENTION

The solution according to the invention comprises an extract product, i.e. more concisely an extract, and a method for producing said extract. The solvent in the extraction process is alkaline ash extract, such as birch ash extract, the ash extract having a pH value of higher than 7, preferably higher than 12.

In the extraction process the solvent can also be water and/or alcohol and/or a water-alcohol mixture, which can be used separately or which can be mixed into the birch ash extract.

The extract product, i.e. extract, obtained as a result of the extraction process is alkaline and the extract contains at least constituents of the ash extract used as a solvent.

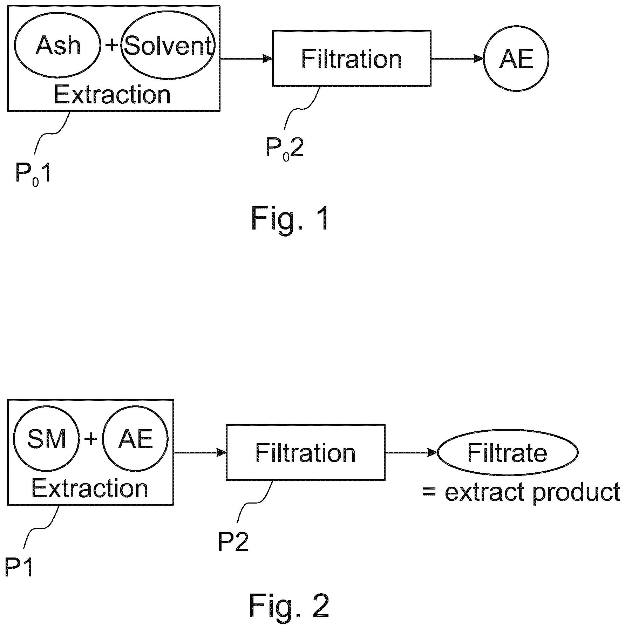

In the method according to the invention preferably alkaline ash extract AE, e.g. birch ash extract, is used as the solvent of the extraction process, in the production of which water, a water-alcohol mixture or birch sap, or a combination of them, is preferably used as a solvent. The general production method for the ash extract AE is presented in Fig. 1. In the first phase P₀1, ash is extracted in a solvent, such as in water. Approx. 6-25 % by mass, suitably 10-18 % by mass and preferably approx. 14 % by mass of ash is used with respect to the amount of solvent. In the second phase P₀2, the solid matter is filtered out of the mixture.

When using birch ash extract as an extraction solvent of starting materials, the minerals in the birch ash extract that is the solvent are obtained in the extract product, in addition to components of the starting material. Owing to the alkalinity of the solvent, the desired minerals and other components, such as phenolic compounds, are recovered from the starting materials more efficiently in the extraction method according to the invention. Phenolic compounds include, *inter alia,* flavonoids, tannins, lignans and phenolic acids.

It is advantageous also to mix alcohol and/or ascorbic acid, citric acid or sodium citrate into the ash solvent for improving, *inter alia,* the solvent power of the extraction process and the preservability of the mixture. The preservatives can be added either before the extraction, during the extraction process and/or after the extraction process.

The general extract production method according to the invention is presented in simplified form in Fig. 2. In the first phase P1, the starting materials SM are dissolved in ash extract AE and in the second phase P2 the solid matter is filtered out of the mixture. The filtrate thus obtained can, as such, already be the desired end extract product, but it can also if necessary be further processed into different extract products. Preferably, the starting materials SM, such as various plants and/or parts of plants, e.g. the leaves of plants, as well as roots and berries, fungi, polypores, *et cetera,* are dissolved in essentially pure birch ash extract, which can also be mixed into water or alcohol, or a mixture of them. In the lattermost case, the starting materials are dissolved in birch ash extract, into which extract water and/or alcohol has been mixed.

According to one preferred embodiment, the starting materials are dissolved in birch ash extract, which has been produced by using birch sap as a solvent, and in which therefore are components from the birch sap.

According to a second preferred embodiment, the starting materials are first dissolved in birch ash extract. After this, the starting materials are filtered and dissolved in water and/or alcohol and/or some other solvent suitable for the purpose. After this, filtration is again performed and the filtrates obtained are combined.

According to another preferred embodiment, the starting materials are first dissolved in water and/or alcohol. After filtering, they are dissolved in birch ash extract and/or some other solvent suitable for the purpose. After this, the filtration is again performed and the filtrates obtained are combined.

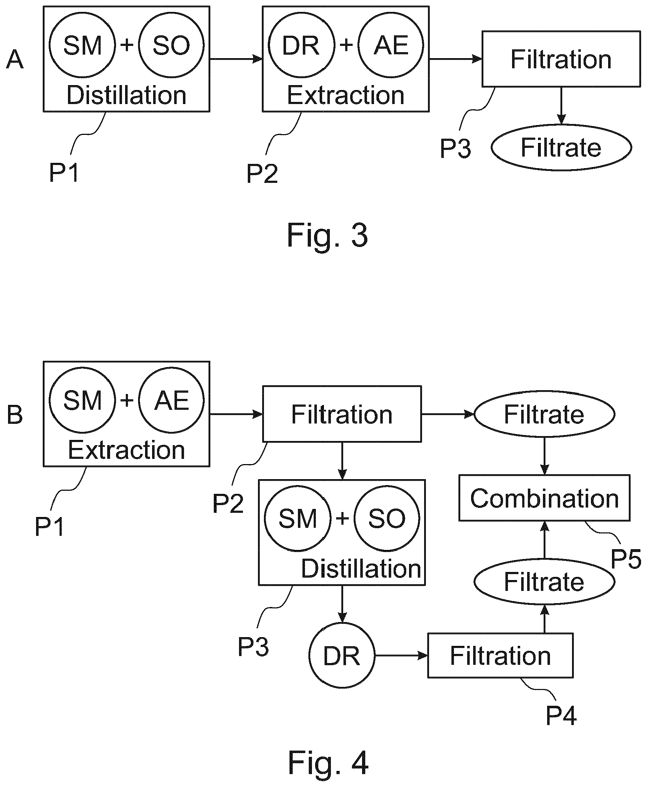

The extraction of the starting materials with ash extract, alcohol, water or a mixture of them can advantageously be performed also by distilling a mixture of solvent and starting materials, until the solvent has almost completely evaporated. Figs. 3 and 4 present simplified extraction processes, as one part of which distillation is performed. In the case of Fig. 3, i.e. in method A, as one part of the extraction process distillation is performed in such a way that in the first phase P1 the starting materials SM are distilled in the solvent SO, such as in a water-alcohol mixture, until the solvent is almost finished, after which the distillation residue DR is extracted in the second phase P2 with the ash extract AE. In the third phase P3, the solid matter is filtered out of the mixture and the filtrate thus obtained is the desired extract, which can also be further processed if necessary. In the case of Fig. 4, i.e. in method B, as one part of the extraction process distillation is performed in such a way that in the first phase P1 the starting materials SM are extracted with ash extract AE. In the second phase P2, the liquid is filtered from the mixture and after this the starting materials SM are distilled in phase P3 in new solvent LI, such as in a water-alcohol mixture. In phase P4, the solid matter is filtered out of the distillation residue DR. In the final phase P5, the filtrates are combined. In this case, the filtrate obtained in phase P4 is mixed into the filtrate obtained in phase P2. The mixture thus obtained is the desired extract, which can also be further processed if necessary.

Yet another preferred method is to keep the ash extract, i.e. ash lye, to be used as a solvent, either on its own or with the starting materials, in wooden barrels so long that components from the barrels, such as various minerals, flavors, lignans and tannins, dissolve in the liquid in the barrel. The time can be from a minute to many years and this can be performed before the start of the extraction process and/or during the extraction process. The ash extract can be cold or boiling hot when placed in a wooden barrel.

In this case, preferably e.g. one or more of the following can be in the wooden barrel: a) wood ash extract, i.e. wood ash lye, as a solvent, b) wood ash extract-alcohol mixture as a solvent, c) wood ash extract as a solvent, in the extraction phase of which tree sap was used as a solvent, d) wood ash extract-alcohol mixture as a solvent, in the wood ash extraction phase of which mixture tree sap was used as a solvent, e) any solvent whatsoever in items a) - d), or a mixture of them, and also any starting material whatsoever mentioned earlier in this description, or combinations of them, placed into this liquid, f) filtered extract produced by extracting from the starting materials. The alcohol can be e.g. wine. Approx. 3-20 % by mass of starting materials are used with respect to the amount of liquid.

In the case of item f), the prepared extract is placed in a wooden barrel to develop further, in which phase components, such as tannins, from the wooden barrel are extracted into the extract. The solvent liquids or extracts in the wooden barrel extract the substances in the wood material of the barrel over time and develop into different solvents or extracts, as in solutions known in the art.

Different solvents and different mixtures of them, a different temperature and time dissolve the various components from barrels made from different wood species. Sometimes there must be also e.g. alcohol and/or citric acid or ascorbic acid in a mixture so that the mixture does not spoil.

Preferably the wooden barrels used in the extraction process according to the invention can be burnt when extract substances no longer leave them, and dried, after which the ash obtained in this way can be further refined and used e.g. in the production of a new type of wood ash solvent.

To accelerate the production of extract, the temperature of the solvent and/or other components to be used in the production is raised. In this case, e.g. the solution, which contains all, or at least most, of the substances needed for the extraction process, and the solvent are heated to the desired temperature and kept at this temperature for the specified time, and after that the solution is allowed to cool. After this, final procedures, such as filtration and the addition of possible additives, are performed for finalizing the extract produced as the end product.

Also essential oils or other finished and commercially available extracts can be mixed into the extract to be produced according to the invention. The essential oils can be e.g. pine, juniper, rose and/or mint essential oils. Generally the oils are strongly diluted before adding. The dilution can be performed e.g. in wine or some other alcoholic liquid, or in some other solvent referred to in the present application, or into a mixture of them, by adding 1 drop of essential oil to 300 ml of solvent. In this case, the mixing can be performed e.g. during the aforementioned extraction process or after it. The extracts can be mixed together also when dry, i.e. as a powder.

In the method according to the invention the ash extract to be used as a solvent and the extract product according to the invention can be produced e.g. in the manners presented below.

### Production of ash extract to be used as a solvent

### Example 1: Shaking method

1) 100 ml (approx. 70 g) of ash is placed in an alkali-proof vessel having an airtight lid;
2) 500 ml of boiling (100 °C) water is poured over the ash and the lid of the vessel is immediately closed;
3) the vessel is shaken vigorously in such a way that the mixture froths;
4) the shaking is repeated many times, e.g. five times;
5) the mixture is allowed to cool and the ash is allowed to settle on the bottom of the vessel;
6) the clear liquid is siphoned off with a thin hose and recovered;
7) the remaining mixture is filtered and the liquid is combined with the liquid obtained in the previous step.

### Example 2: Boiling in water method

1) 100 ml (approx. 70 g) of ash is heated in 500 ml water under a lid for approx. 12 minutes, e.g. in a stainless steel pot;
2) the mixture is allowed to cool and the ash is allowed to settle on the bottom of the vessel;
3) the clear liquid is siphoned off with a thin hose and recovered;
4) the remaining mixture is filtered and the liquid is combined with the liquid obtained in the previous step.

### Example 3: Boiling in birch sap

1) 100 ml (approx. 70 g) of ash is heated in 500 ml of birch sap at approx. 55-100 °C either under a lid or without a lid for from a minute to many hours;
2) the mixture is allowed to cool and the ash is allowed to settle on the bottom of the vessel;
3) the clear liquid is siphoned off with a thin hose and recovered;
4) the remaining mixture is filtered and the liquid is combined with the liquid obtained in the previous step.

### Example 4: Distillation method

1) 100 ml (approx. 70 g) of ash is heated in 500 ml water in a pressure distillation apparatus at 5 bar pressure and 150 °C, until the liquid is almost finished;
2) the cooled mixture is filtered and the liquid recovered.

### Example 5: Vacuum filtration method

1) 100 ml (approx. 70 g) of ash is placed in a Büchner funnel provided with very thin filter paper, which funnel is placed in a suction bottle connected to a vacuum pump;
2) 100 ml of hot water at 80-100 °C is conducted through the filter by means of suction pressure;
3) the addition of water is repeated many times, e.g. five times, either with the same or new water;
4) the filtrates obtained are recovered.

### Extraction of starting materials

### Example 1: Solution method at room temperature

1) 25 g of fresh rose root is pulverized and placed in an alkali-proof vessel, such as a glass vessel;
2) 250 ml of ash extract is added as solvent;
3) possibly one drop of e.g. diluted essential oil of mint is added;
4) the mixture is allowed to stand at room temperature for approx. 48 hours;
5) the clear liquid is siphoned off with a thin hose and recovered;
6) the remaining mixture is filtered and all the liquid is pressed out of the starting material;
7) the liquids thus obtained are combined and recovered.

### Example 2: Stewing method

1) 25 g of dried birch buds are pulverized and placed in an alkali-proof having an airtight lid;
2) 400 ml of ash extract is added as solvent and possibly a drop of e.g. essential oil of juniper;
3) the mixture is heated for 2 hours at 60 °C under a lid, e.g. in an oven;
4) the mixture is allowed to cool;
5) the clear liquid is siphoned off with a thin hose and recovered;
6) the remaining mixture is filtered and all the liquid is pressed out of the starting material;
7) the liquids thus obtained are combined and recovered.

### Example 3: Distillation method

1) 25 g of pulverized birch bark is heated in 400 ml of ash extract in a vacuum distillation apparatus or in a vacuum rotary evaporator at 0.5 bar pressure and at 80 °C until the solvent is almost finished;
2) the cooled mixture is filtered and the liquid recovered.

### Example 4: Wooden barrel method

1) 250 g of dandelion root is pulverized and placed in a five-liter oak barrel;
2) two liters of ash extract and two liters of wine are added as a solvent;
3) possibly approx. 1% by mass of citric acid is added;
4) the mixture is allowed to stand at room temperature for approx. 12 months;
5) the mixture is filtered and the liquid recovered;

### Final procedures for finalizing the extract as an end product

Example 1:
1) approx. 0.5 % by mass of ascorbic acid is added as a preservative;
2) one drop of essential oil of pine diluted with wine (1 drop / 300 ml of wine) is added per 300 ml of finished extract;
3) the extract is packed in small, tightly closed receptacles, such as 50 ml glass bottles, in sterile conditions.

Example 2:
1) approx. 2% by mass of sodium citrate is added as a preservative;
2) the extract is packed in small, tightly closed receptacles, such as 50 ml glass bottles, in sterile conditions.

It is obvious to the person skilled in the art that different embodiments of the invention are not limited to the examples described above, but that they may be varied within the scope of the claims presented below. Thus, for example, the alkaline solvent can be extract made from some other ash than birch ash. In this case, the solvent is e.g. alkaline ash extract, which has been produced from ash from other trees than birch and/or from ash originating from the burning of various plants and/or from superterranean parts and/or subterranean parts of bushes. In this case, the mineral concentrations of both the solvent and the produced extract vary, depending on the soil and the material used for producing the ash.

It is also obvious to the person skilled in the art that also other water than spring water or tap water can be used in the production of the ash extract to be used as a solvent. For example, distilled water can be used in the production of the ash extract used as a solvent, in which case the ash extract is different in composition than e.g. ash extract made in spring water or tap water. The ash extract to be used as a solvent, i.e. wood ash lye, can be produced also from alkalized water, which is already then alkaline. Some alkali can also be added afterwards to the ash extract to be used as a solvent. Some other alkaline solvent than ash extract can also be used as a solvent.

It is further obvious to the person skilled in the art that in the shaking method described above for producing ash extract to be used as a solvent the shaking can also be performed mechanically with a continuous or intermittent motion. The temperature of the water can also be some other temperature than 100 °C. The shaking can also be continued after the liquid has cooled in the ways mentioned above. The shaking can also be performed for from approx. a minute to many days. During shaking, negative pressure develops in the vessel when hot liquid cools in the airtight vessel. The negative pressure can also be brought about mechanically. The shaking can also be performed in a type of vessel in which the mixture can be warmed again to become hot, e.g. in an oven, and can be shaken now and then in the aforementioned ways. Heating and cooling can be repeated many times.

It is further obvious to the person skilled in the art that in the examples 1-3 described above for producing ash extract to be used as a solvent, the mixture can also be filtered immediately after shaking or boiling, without siphoning, and the liquid recovered.

It is also obvious to the person skilled in the art that in the examples 1-5 described above for producing ash extract to be used as a solvent, the solvent can also be e.g. wine or some other alcoholic liquid. The alcohol can be allowed either to evaporate or to cool into the finished extract.

It is further obvious to the person skilled in the art that other tree sap than birch sap can be used in the production of the ash extract used as a solvent. Other such trees are e.g. other deciduous trees, such as maple, oak and alder. Boiling ash in sap can also be performed in a vessel in which the heating resistor is inside the vessel. Production of the ash extract to be used as a solvent in sap can also be performed by shaking ash and hot sap in an airtight vessel, as in Example 1 for producing the ash extract to be used as a solvent.

It is again obvious to the person skilled in the art that production of the ash extract to be used as a solvent by distillation can be performed also with other methods than high-pressure distillation.

It is also obvious to the person skilled in the art that the ash extract to be used as a solvent and other extracts can also be produced in a pressurized vessel such as an autoclave, in which case the production is more efficient.

It is further obvious to the person skilled in the art that the amount of ash in all the aforementioned examples for producing ash extract to be used as a solvent can also be other than 1 part by volume of ash to 5 parts by volume of liquid. Additionally, ash extract can also be produced by simmering an ash-liquid mixture e.g. in an oven or in a vessel provided with electric resistance at 55-120 °C for from a minute to many hours or days. The vessel can be either closed or open, and there can be high pressure or negative pressure in the vessel. The boiling time or simmering time can be from a minute to many days. The mixture can also be stirred from time to time. Ash extract can also be produced in vessels of the liquidizer type by placing ash and hot liquid e.g. in a liquidizer and allowing it to blend for from approx. a min to many hours. The mixture can be heated sometimes and then the mixing process continued.

It is also obvious to the person skilled in the art that also tree sap or alcohol, such as wine, or mixtures of them in different proportions, can be used as a solvent in addition to, or instead of, ash extract when extracting starting materials. The extraction process can also be performed with the shaking method or vacuum filtration method or in a liquidizer, such as the production of ash extract.

It is also obvious to the person skilled in the art that with the solution according to the invention also very functional homeopathic products can be produced for various purposes, depending on the starting materials. Likewise, with the solution according to the invention also spagyric products and also new types of finished products can be produced, by combining the production method according to the invention with production methods already known in the art. Likewise, the use of products produced with the alkaline extraction method can also be further developed.

It is also obvious to the person skilled in the art that with the solution according to the invention even more different products can be produced from different starting materials and with different method phases.

For example, not necessarily enough of the desired substances dissolve from all starting materials, such as from fungi, roots, polypores and various hard materials, in ash extract. In such a case, the starting materials with the ash solvent, or placed in completely new solvent, can be distilled, in which case the distillation residue thus obtained can after filtering be the end product to be produced. Filtered distillation residue can also be dried, in which case it can be used as a dried extract product. In addition, products can also be produced in which the aforementioned distillation residue is further added to the extract liquids obtained from dissolving. The extracts, distillation residues and products thus obtained can also be combined in other ways and with other substances.

In the solution according to the invention, it can in all cases also be performed in such a way that the starting materials, such as plants, roots, polypores and fungi, are dried either before or after extraction and burnt into ash, and the extract made from which ash can be used as a solvent in a new extraction process, on its own or mixed with other solvents, or it can be added to finished extracts. In the final extract product obtained in this way are also the minerals contained in the starting materials.

It can also be performed in such a way that after the extraction process according to the invention, in which extraction process ash, preferably wood ash, such as birch ash, is used in the solvent at least as one component, the starting materials, such as plants, roots, polypores and fungi, that were in the solvent and that can be dried are dried and burnt into ash. After this, new ash extract can be produced from this ash, which new ash extract can be used in different ways, e.g. as a solvent in extracting different starting materials or for some other purpose.

The wood ash solvent to be used in the solution according to the invention, i.e. wood ash lye, can also be a desired end product as such. Wood as lye can also be made as a dry extract, from which different products can be further developed.

It is also further obvious to the person skilled in the art that with the solution according to the invention all the extracts obtained can be produced to become dry products, in which case extract products can be e.g. absorbed into pills, the use of which is extremely easy.

It is also obvious to the person skilled in the art that in the solution according to the invention, in addition to the aforementioned starting materials, also parts of other plants as well as the sap of other trees can be used as starting materials, such as the sap of the Boswellia serrata plant, spices, nuts, seeds, cones, pollen, lecithins, the bark of trees, birch bark, hard and dry bark, parts of inner bark, and plants growing in water as well as their roots, propolis produced by bees, honey, spiders' webs, and generally all the materials obtainable from nature that withstand the alkalinity of the ash solvent to be used in the solution according to the invention.

It is further obvious to the person skilled in the art that the extract product produced with the solution according to the invention can also be flavored. The flavoring can be performed with e.g. stevia, rosewater and/or with various natural or artificial flavorings, or combinations of these. Additionally, also various useful bioactive compounds, such as vitamins and various minerals, can be added to the extracts. These additions can be made in any process phase whatsoever, i.e. before extraction, during the extraction process, and after the extraction process. When this type of extract product is used as a liquid, vitamins are absorbed e.g. into a person's organs very efficiently and quickly. Carbonated or uncarbonated soft drinks can also be produced from the extract product.

It is also obvious to the person skilled in the art that all the extraction methods and starting materials presented in this description can be mixed with each other and further developed. Furthermore, all the liquids used in the solution according to the present invention, i.e. solvents and/or finished extract liquids, can also be distilled when they have been in a wooden barrel for the desired time.

## Claims

1. Extract produced by means of an alkaline solvent, wherein the starting materials are organic materials or biogenic materials, **characterized in that**, in addition to the substances dissolved from the starting materials, the extract contains components, such as minerals, contained in the ash extract used as a solvent, and **in that** 3-20 % by mass, suitably 6-15 % by mass and preferably approx. 10 % by mass of starting materials are used with respect to the amount of ash extract used as a solvent, and **in that** the extract has been made by dissolving the starting materials in ash extract at 4-120 °C for from a minute to two years, which ash extract has been produced by extracting ash in a solvent, the temperature of the solvent being between 55-150 °C, by using more than 5 % by mass ash with respect to the amount of solvent, and which ash extract comprises both components of ash and also solvent used in producing the ash extract.

2. Extract according to claim 1, **characterized in that** the ash extract used as a solvent is an ash extract produced from ash of botanical or fungal origin by boiling, the ash extract having a pH value of higher than 7, preferably higher than 12, and **in that** one part by volume of ash has been boiled in five parts by volume of solvent, such as water and/or tree sap and/or alcohol, for 10-15 minutes, preferably for 12 minutes.

3. Extract according to claim 1 or 2, **characterized in that** the ash extract used as a solvent is an alkaline birch ash extract, and **in that** the extract produced is an alkali.

4. Extract according to any of the preceding claims 1, 2 or 3, **characterized in that** the extract contains components contained in birch sap and/or in the sap of some other deciduous tree used as a solvent in producing the ash extract used as a solvent.

5. Extract according to any of the preceding claims 1-4, **characterized in that** the extract contains components, such as tannins, from the wooden barrels in which the ash extract used as a solvent has been stored with or without the starting materials before the start of the extraction process.

6. Extract according to any of the preceding claims 1-5, **characterized in that** vitamins, flavorings and/or essential oils are added to the extract.

7. Method for producing an extract, in which method the starting materials are organic materials or biogenic materials and an alkaline solvent is used as the solvent, **characterized in that** an ash extract of botanical or fungal origin is used as the solvent, and **in that** 3-20 % by mass, suitably 6-15 % by mass, and preferably approx. 10 % by mass of starting materials are used with respect to the amount of ash extract to be used as a solvent, and **in that** the extract is made by dissolving the starting materials in ash extract at 4-120 °C for from a minute to two years, which ash extract has been produced by extracting ash in a solvent, the temperature of the solvent being between 55-150 °C, by using more than 5 % by mass of ash with respect to the amount of solvent, and which ash extract comprises both ash and also solvent used in producing the ash extract.

8. Method according to claim 7, **characterized in that** the starting materials are dissolved in an ash extract of botanical or fungal origin, the extract having a pH value of higher than 7, preferably higher than 12, and **in that** the ash extract has been prepared by boiling one part by volume of ash extract in five parts by volume of solvent, such as water and/or tree sap and/or alcohol, for 10-15 minutes, preferably for 12 minutes.

9. Method according to claim 7 or 8, **characterized in that** the starting materials are dissolved in undiluted birch ash extract or in birch ash extract into which water and/or alcohol has been mixed.

10. Method according to any of the preceding claims 7, 8 or 9, **characterized in that** vitamins, flavorings and/or essential oils are mixed into the extract being prepared.

11. Method according to any of the preceding claims 7-10, **characterized in that** as one part of the extraction process distillation is performed in such a way that the starting materials and solvent are distilled at first, or the starting materials are distilled after extraction and filtration, separately in a new solvent, and **in that** the distillation residue is processed in one or more of the following ways: a) the distillation residue obtained is prepared as such to be the end product, b) the distillation residue obtained is extracted again in a second solvent in which preferably are components of ash extract, c) the distillation residue obtained is filtered and mixed into the filtrate obtained in the extraction phase.

12. Method according to any of the preceding claims 7-11, **characterized in that** the extraction process, or a part of it, is performed by distilling in such a way that the starting materials placed into the solvent and the solvent are distilled in negative pressure until the solvent is almost finished, and **in that** approx. 6 % by mass of starting materials are used with respect to the amount of solvent, such as ash extract.

13. Method according to any of the preceding claims 7-12, **characterized in that** the extraction process is performed in two phases in such a way that in the first dissolving phase birch ash extract is used as a solvent of the starting materials, and in the second dissolving phase the solvent is water and/or alcohol and/or some other solvent suitable for the purpose, or in such a way that in the first dissolving phase water and/or alcohol is used as a solvent of the starting materials, and in the second dissolving phase the solvent is birch ash extract and/or some other solvent suitable for the purpose, and **in that** preferably after each dissolving phase filtration is performed and finally the filtrates are combined.

14. Method according to any of the preceding claims 7-13, **characterized in that** the ash extract used as a solvent, as such or with the starting materials to be used in the extraction process, is stored in a wooden barrel before the start of the extraction process and/or during the extraction process.

15. **Use** of ash extract as a solvent for extracting starting materials, the starting materials being organic materials or biogenic materials, and which ash extract has been produced by extracting ash in a solvent, the temperature of the solvent being between 55-150 °C, by using more than 5 % by mass ash with respect to the amount of solvent, and which ash extract comprises both ash of botanical or fungal origin and also solvent used in producing the ash extract, such as water and/or tree sap and/or alcohol.
